# EUROPÄISCHE PATENTSCHRIFT

(11) **EP 1 417 981 B1**
(45) Veröffentlichungstag und Bekanntmachung des Hinweises auf die Patenterteilung: **30.01.2013**
(21) Anmeldenummer: 03023860.4
(22) Anmeldetag: 21.10.2003
(51) Int. Cl.: A61M 5/315

(54) **Medizinische Spritze**
Medical Syringe
Seringue medicale

(30) Priorität: 11.11.2002 DE 10252220
(43) Veröffentlichungstag der Anmeldung: 12.05.2004
(73) Patentinhaber: Schott AG, 55122 Mainz (DE)
(72) Erfinder: Koller, Horst, Dipl.-Ing., 9032 Engelburg (CH); Steigenberger, Markus, 65474 Bischofsheim (DE); Mosimann, Karl, 9514 Wuppenau (CH); Kadur, Thomas, Boyle, MS 38730 (US)
(74) Vertreter: Fuchs

(56) Entgegenhaltungen:
- DE-A- 4 331 137
- DE-C- 331 326
- US-A- 3 747 812
- US-A- 4 072 149
- US-A- 4 267 846
- US-A- 4 711 637
- US-A- 5 084 017
- US-A- 5 997 511
- US-B1- 6 296 625

## Beschreibung

Die Erfindung bezieht sich auf eine medizinische Spritze, bestehend aus
- einem Spritzenzylinder aus Glas oder Kunststoff mit angeformten Spritzenkopf, in welchem entweder eine Injektionskanüle integriert ist oder der einen Luer-Anschlusskonus besitzt, und mit einer Griffplatte am offenen Ende sowie
- einem elastomeren Kolbenstopfen, der in den Spritzenzylinder einbringbar ist, und der ein SacklochGewinde für das Einschrauben einer Kolbenstange mit kreuzartigem Querschnitt aufweist, und
- einem Anschlagelement zum Verhindern eines unbeabsichtigten Herausziehens des Kolbenstopfens.

Für die parenterale Verwendung von flüssigen pharmazeutischen Produkten ist es bekannt, Ampullen und Injektionsflaschen zu verwenden. Für die Injektion der in diesen Flaschen enthaltenen Produkte wird eine mit der entsprechenden Injektionskanüle kombinierte medizinische Spritze benötigt. Das bedeutet, dass das flüssige pharmazeutische Produkt vor seiner endgültigen Verwendung in die medizinische Spritze überführt werden muss. Dieses Verfahren ist nicht nur zeitraubend, sondern es ermöglich auch eine große Anzahl von Verunreinigungsquellen.

Um eine sichere Verwendung von pharmazeutischen Produkten sicherzustellen, sind vorgefüllte Einmalspritzen auf dem Markt. Sie erlauben eine schnelle Injektion des enthaltenden Produktes nach relativ einfacher Handhabung. Derartige vorgefüllte Spritzen weisen einen Spritzenzylinder aus Glas oder Kunststoff mit angeformten Spritzenkopf, in welchem entweder eine Injektionsnadel integriert ist oder der einen Luer-Anschlußkonus einer Kegelverbindung, gegebenenfalls einer verriegelbaren Kegelverbindung (Luer-Lock) besitzt, auf. An dem anderen, offenen Ende des Spritzenzylinders ist eine Fingergriffplatte angebracht, entweder einstückig angeformt oder als separates Teil aufgesteckt, wobei das offene Ende des Spritzenzylinders mit einem elastomeren Kolbenstopfen verschließbar ist, der eine Gewinde-Sackbohrung aufweist, in welche im Applikationsfall eine Kolbenstange mit einem kopfseitigen Gewinde einschraubbar ist. Die vorgenannten Einmalspritzen, auch Fertigspritzen genannt, mit Spritzenkörpern aus Glas, sind Gegenstand der Norm DIN ISO 11040, wobei beispielsweise der Spritzenzylinder im Teil 4 und der elastomere Standardkolbenstopfen sowie die Standard-Kolbenstange aus Kunststoff mit kreuzförmigem Querschnitt im Teil 5 beschreiben sind.

Vorgefüllte Einmalspritzen aus Kunststoff sind ebenfalls Bestandteil von Normenentwürfen und in zahlreichen Veröffentlichungen beschrieben, und haben insbesondere, was den Kolbenstopfen und die Kolbenstange anbelangt, einen gleichartigen Aufbau.

Von Spritzen der vorgenannten Art, die sich am Markt durchgesetzt haben und im großen Umfang benutzt werden, geht die Erfindung aus. Sie ist jedoch auch anwendbar bei nicht vorgefüllten Spritzen, die, wie eingangs erwähnt, beim Applizieren zunächst aus einem Fläschchen aufgezogen werden.

Um eine problemlose Anwendung der vorgenannten Spritzen zu gewährleisten, sind neben dem vorbeschriebenen Grundaufbau weitere konstruktive Ausgestaltungen notwendig.

Um während der Aufbewahrung, Vorbereitung und/oder Anwendung vorgenannter Spritzen ein unbeabsichtigtes Herausziehen des Kolbenstopfens aus dem Spritzenzylinders zu verhindern, bzw. um zu gewährleisten, dass auch der oberste Dichtring des Kolbenstopfens immer im Spritzenzylinder bleibt, ansonsten die Keimdichtheit der vorgefüllten Spritze nicht mehr gewährleistet werden könnte, ist es notwendig, einen sogenannten Backstop an dem Spritzenkörper anzubringen, typischerweise durch einen den Durchmesser des Spritzenzylinders an seinem offenen Ende einengenden Anschlag.

Der Stand der Technik zeigt verschiedene Lösungen für diesen Anschlag.

So ist bei der bekannten Einmalspritze nach der DE 100 36 829 A der Anschlag durch eine ringförmige Einengung des Spritzenzylinders ausgebildet, die, um das Einbringen des Kolbenstopfens nach der Befüllung der Spritze nicht zu behindern, nach dem Einbringen des Kolbenstopfens in den gefüllten Spritzenzylinder angebracht wird.

Die DE 43 31 137 A sieht ein am griffplattenseitigen Ende der Spritze an einer Fingerauflage mit vergrößernder Auflagefläche gegenüber der DIN-Griffplatte angebrachten Anschlagelement vor, das mindestens einen in den Spritzenzylinder hineinreichenden Vorsprung aufweist, gegen den der Kolbenstopfen bei einem axialen Verschieben in Richtung des offenen Endes des Spritzenzylinders anstößt, wodurch ein ungewolltes Herausziehen des Kolbenstopfens aus dem Spritzenzylinder verhindert wird. Dieses bei der bekannten Spritze als Vorsprung ausgebildete Anschlagelement, welches einstückig mit einer Fingerauflage ausgestaltet ist, wird auf den Spritzenkörper seitlich aufgeklemmt, wobei im montierten Zustand der Vorsprung innenliegend an der Wandung des Spritzenkörpers anliegt. Dabei besteht jedoch die Gefahr, dass aufgrund des Aufklippens des Anschlagelementes und der damit einstückig ausgebildeten Fingerauflage auf dem Spritzenkörper keine ausreichende Fixierung der Fingerauflage sichergestellt ist, so dass dementsprechend insbesondere auch während des Injiziervorganges die Spritze vom jeweiligen Anwender nicht sicher gehandhabt werden kann und dementsprechend auch verrutschen kann.

Die US 5, 700, 247 zeigt eine Spritze, die eine Fingerauflage mit integrierter Backstop-Funktion aufweist. Diese Fingerauflage ist ein separates Spritzgußteil, das aus einer Boden- und Deckelplatte besteht, welches von der Seite aus auf die entsprechende DIN-Griffplatte des Spritzenkörpers aufgeschoben wird. Die im Durchmesser verkleinerte Bodenplatte dient als Backstop-Funktion, die verhindert, das der Kolbenstopfen versehentlich aus der Spritze gezogen wird.

Die US 4,909,788, und hierzu ähnlich die US 5,554,133, offenbart eine Spritze mit einem elastomeren Kolbenstopfen, welcher am offenen Ende eine Griffleiste besitzt. Von diesem offenen Ende her ist eine vergrößerte Fingerauflage aufschiebbar, rastet in die besagte Griffleiste ein, und befindet sich anschließend im Passsitz, wobei sie um die Spritzenachse drehbar ist.

Die US 4,072,149 zeigt eine Spritze mit einer Kunststoffgriffleiste, welche sich in den Spritzenkörper hinein erstreckt und gleichzeitig ein Anschlagselement für den Kolbenstopfen bildet.

Neben dieser Backstop-Funktion kommt es bei Spritzen auch darauf an, dass die Kolbenstange gegen ein Verdrehen gesichert ist, damit sie sich nicht ungewollt beim Applizieren herausdrehen kann. Bei dem Vorgang des sogenannten Aspirierens, bei dem der Kolbenstopfen mehrfach hin- und herbewegt werden muss, zum Beispiel beim Applizieren einer wässrigen Lösung in einen Behälter mit einer gefriergetrockneten pharmazeutischen Substanz, kann es passieren, dass sich die Kolbenstange aus dem Gewinde im Kolbenstopfen löst. Eine derartige Verdrehsicherung wird jedoch im vorstehend zitierten Stand der Technik nicht angesprochen, wohl aber in der US 4,711,637. Bei der letztgenannten Schrift ist eine Kolbenstange mit kreuzartigem Querschnitt vorgesehen, für die eine Verdrehsicherung bestimmt ist.

Der Erfindung liegt die Aufgabe zugrunde, ausgehend von der eingangs bezeichnenden Spritze diese so auszubilden, dass auf einfache Weise eine Backstop-Funktion hinsichtlich des Kolbenstopfens und eine Verdrehsicherung hinsichtlich der Kolbenstange erzielbar sind.

Die Lösung dieser Aufgabe gelingt bei einer medizinischen Spritze, bestehend aus
- einem Spritzenzylinder aus Glas oder Kunststoff mit angeformten Spritzenkopf, in welchem entweder eine Injektionskanüle integriert ist oder der einen Luer-Anschlusskonus besitzt, und mit einer Griffplatte am offenen Ende sowie
- einem elastomeren Kolbenstopfen, der in den Spritzenzylinder einbringbar ist, und der ein SacklochGewinde für das Einschrauben einer Kolbenstange mit kreuzartigem Querschnitt aufweist, und
- einem Anschlagelement zum Verhindern eines unbeabsichtigten Herausziehens des Kolbenstopfens,

gemäß der Erfindung dadurch, dass ein von der Kopfseite des Spritzenzylinders her auf die Griffplatte aufsteckbares Kunststoffteil vorgesehen ist, das einen Abschnitt für die Halterung an dem Spritzenzylinder und einen sich zur Griffplatte hin unter Bildung einer vergrößerten Fingerauflage erweiternden haubenartigen Abschnitt aufweist, an dem gegenüberliegend im Bereich des offenen Endes des Spritzenzylinders zwei Schnapphaken einstückig angeformt sind, derart, dass sie beim Aufstecken sich über die Griffplatte schieben und an der Innenseite des Spritzenzylinders mit geringer Eintauchtiefe einschnappen.

Durch die erfindungsgemäßen Maßnahmen kann mittels eines einzigen, einfachen Kunststoffteiles sowohl eine Verdrehsicherung der Kolbenstange mit kreuzförmigem Querschnitt als auch die Backstop-Funktion erzielt werden, d. h. dieses Kunststoffteil stellt eine Verdrehsicherung von Kolbenstangen mit kreuzförmigen Querschnitt mit integrierter Backstop-Funktion dar. Die Schnapphaken verringern nämlich an zwei Stellen den Durchmesser des Spritzenzylinders an seinem offenen Ende, so dass eine Drehung der Kolbenstange infolge ihres kreuzartigen Querschnittes mit einer Füllkurve, die dem Innendurchmesser des Spritzenzylinders angepasst ist, nicht mehr als 90° möglich ist. Zugleich dienen die eintauchenden Schnapphaken als Anschlagelement zum Verhindern des Herausziehens des Kolbenstopfens. Da die Schnapphaken nur eine geringe Eintauchtiefe in den Spritzenzylinder haben, wird die an der Zylinderinnenwand typischerweise angebrachte Silikonisierung nicht beeinträchtigt.

Vorzugsweise ist das Kunststoffteil einstückig ausgebildet. Prinzipiell kann es jedoch auch aus mehreren Einzelkomponenten bestehen.

Für die Fertigung des Kunststoffteiles bzw. dessen Einzelkomponenten kommen alle konventionellen Formgebungsverfahren in Betracht.

Da die vorgenannten medizinischen Spritzen einen Massenartikel darstellen, kommt es sehr auf die Herstellungskosten dieser Spritze an. Die zusätzlichen Kosten für das separat herzustellende Kunststoffteil können gering gehalten werden, wenn das Kunststoffteil ein Spritzgußteil ist, was in großen Serien mit geringen Kosten herstellbar ist.

Ist die Griffplatte nach DIN ISO 11040 als schmale Leiste ausgebildet, dann ist gemäß einer Ausgestaltung der Erfindung der sich zur Griffplatte hin erweiternde haubenartige Abschnitt des Kunststoffteiles an der Basis ebenfalls als schmale Leiste ausgebildet, deren Breite gleich derjenigen der Griffplatte ist und deren die erweiternde Fingerauflage bildende Länge größer als die der DIN-Griffplatte ist. Dadurch kann mit dem ein- und demselben Kunststoffteil neben der Verdrehsicherung und der Backstop-Funktion auch eine verbesserte Fingerauflage geschaffen werden.

Um eine grifffreundliche Handhabung der Spritze zu ermöglichen, sind dabei die über die DIN-Griffplatte hinaus stehenden Enden der Fingerauflagenleiste abgerundet.

Anhand eines in den Figuren 1 bis 3 in verschiedenen Ansichten dargestellten Ausführungsbeispieles einer normierten vorfüllbaren Spritze mit einem separaten Kunststoffteil als Verdrehsicherung und Backstop-Anschlagelement wird die Erfindung näher erläutert.

Es zeigen:
- Fig. 1: eine schematische Längsschnittdarstellung der Spritze mit einem Schnitt entlang der Ausdehnung der Griffplatte,
- Fig. 2: ebenfalls in einer schematisierten Längsschnittdarstellung die erfindungsgemäß ausgestaltete Spritze mit einer Schnittlinie senkrecht zur Ausdehnung der Griffplatte, d. h. senkrecht zu der Darstellung nach Fig. 1 und
- Fig.3: eine Draufsicht auf das fingerauflagenseitige Ende der Spritze nach Fig. 1.

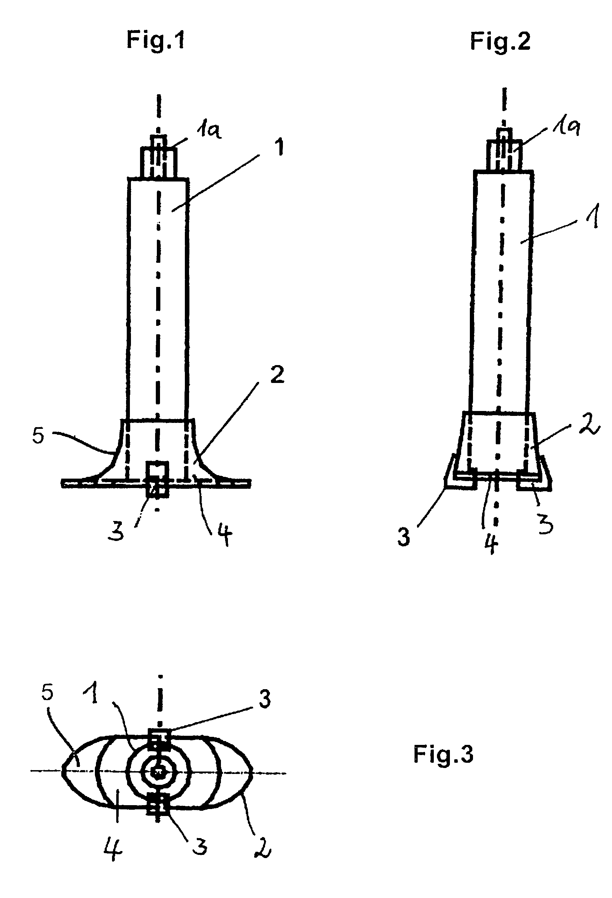

Die Spritze nach den Figuren 1 bis 3 ist eine normierte vorfüllbare Spritze mit einem Spritzenzylinder 1, an dem ein Spritzenkopf 1 a angeformt ist. Im vorliegenden Beispiel ist ein Luer-Konus bzw. ein Luer-Lock für eine dann verriegelbare Kegelverbindung als Anschlusselement angeformt. Es ist jedoch auch möglich, dass in dem Spritzenkopf direkt eine Injektionskanüle integriert ist.

Am gegenüberliegenden offenen Ende des Spritzenzylinders 1 ist eine DIN-Griffplatte 4 einstückig angeformt. Das offenen Ende des befüllten Spritzenzylinders ist dabei in nicht dargestellter Weise mit einem Kolbenstopfen aus elastomerem Material mit äußeren Dichtrippen verschlossen, in den eine Standard-Kolbenstange mit kreuzförmigem Querschnitt und scheibenförmiger Fingerauflage einschraubbar ist. Damit der Kolbenstopfen an der Innenwandung des Spritzenzylinders 1 beim Applizieren gut gleiten kann, ist dieser mit einer Innensilikonisierung versehen.

Soweit der bekannte Grundaufbau einer medizinischen Spritze.

Zur Gewährleistung einer Verdrehsicherung und der Backstop-Funktion sieht die Erfindung ein Kunststoffteil 2 vor, welches einen kopfseitigen Abschnitt für die Halterung an dem Spritzenzylinder und einen sich zur Griffplatte 4 hin unter Bildung einer vergrößerten Fingerauflage erweiternden haubenartigen Abschnitt 5 aufweist. An diesem Abschnitt sind gegenüberliegend im Bereich des offenen Endes des Spritzenzylinders 1 zwei Schnapphaken 3 einstückig angeformt. Der sich zur Griffleiste hin erweiternde Abschnitt des Kunststoffteiles ist angepasst an die DIN-Griffplatte 4 ebenfalls als schmale Leiste ausgebildet, deren Breite gleich derjenigen der Griffplatte 4 ist und deren Länge größer als die der Griffplatte ist, so dass eine erweiterte Fingerauflage gebildet wird, wie insbesondere die Fig. 3 zeigt. Um eine günstige Handhabung der Spritze sicherzustellen, sind dabei die über die DIN-Griffplatte 4 hinausstehenden Enden der Fingerauflagenleiste abgerundet.

Das separate Kunststoffteil 2 ist dabei vorzugsweise ein Spritzgußteil, was mit geringen Kosten herstellbar ist.

Das spritzgegossene Kunststoffteil 2 der Kolbenstange wird von oben, d. h. kopfseitig auf die Spritze nach dem Labelvorgang aufgesteckt. Durch die möglichst geringe axiale Höhe des Kunststoffteiles wird das aufgebrachte Label nicht bedeckt. Die an der Verdrehsicherung angespritzten Schnapphaken 3 werden dabei über die gerade Seite der Griffplatte 4 geschoben und schnappen an der Innenseite des Spritzzylinders ein (s. Fig. 2 und 3). Da die Schnapphaken nur eine geringe Eintauchtiefe haben, wird die an der Zylinderinnenwand angebrachte Silikonisierung mit Vorteil nicht beeinträchtigt.

Die Schnapphaken 3 engen, wie insbesondere die Fig. 2 zeigt, den Innendurchmesser des Spritzenzylinders ein und verhindern eine Verdrehung der Kolbenstange über 90° hinaus. Somit ist gewährleistet, dass auch bei entsprechender Notfallmedikation ein Herausdrehen der Kolbenstange nicht möglich ist.

Die in den Spritzenzylinder hineinragenden stirnseitigen Enden der Schnapphaken dienen als Anschlag für den Kolbenstopfen, d. h. gewährleisten auch die Backstop-Funktion.

Das einfach herzustellende Kunststoffteil 2 erfüllt damit mit Vorteil gleichzeitig drei Funktionen:
- es verhindert ein Rausziehen des Kolbenstopfens,
- es verhindert, dass die Kolbenstange herausgedreht werden kann,
- es stellt eine vergrößerte Fingerauflage dar.

## Patentansprüche

1. Medizinische Spritze, bestehend aus
- einem Spritzenzylinder (1) aus Glas oder Kunststoff mit angeformten Spritzenkopf (1a), in welchem entweder eine Injektionskanüle integriert ist oder der einen Luer-Anschlusskonus besitzt und mit einer Griffplatte (4) am offenen Ende sowie
- einem elastomeren Kolbenstopfen, der in den Spritzenzylinder einbringbar ist und der ein Sacklochgewinde für das Einschrauben einer Kolbenstange mit kreuzartigem Querschnitt aufweist,
**dadurch gekennzeichnet, dass** ein von dem Kopf (1a) des Spritzenzylinders (1) her auf die Griffplatte (4) aufsteckbares Kunststoffteil (2) vorgesehen ist das einen Abschnitt für die Halterung an dem Spritzenzylinder (1) und einen sich zur Griffplatte (4) hin, unter Bildung einer vergrößerten Fingerauflage erweiternden haubenartigen Abschnitt (5) aufweist, an dem gegenüberliegend im Bereich des offenen Endes des Spritzenzylinders zwei Schnapphaken (3) einstückig angeformt sind derart, dass sie beim Aufstecken sich über die Griffplatte (4) schieben und an der Innenseite des Spritzenzylinders (1) mit geringer Eintauchtiefe einschnappen, so dass dadurch ein Anschlagelement zum Verhindern eines unbeabsichtigten Herausziehens des Kolbenstopfens gebildet wird, und eine Verdrehung der Kolbenstange über 90° hinaus verhindert wird.

2. Spritze nach Anspruch 1, **dadurch gekennzeichnet, dass** das Kunststoffteil (2) einstückig ausgebildet ist.

3. Spritze nach Anspruch 1, **dadurch gekennzeichnet, dass** das Kunststoffteil (2) aus mehreren Einzelkomponenten besteht.

4. Spritze nach Anspruch 2 oder 3, **dadurch gekennzeichnet, dass** das Kunststoffteil (2) oder die jeweilige Einzelkomponente ein Spritzgussteil ist.

5. Spritze nach einem der Ansprüche 1 bis 4, **dadurch gekennzeichnet, dass** die Griffplatte (4) als schmale Leiste ausgebildet und der sich zur Griffleiste hin haubenartig erweiternde Abschnitt (5) des Kunststoffteiles (2) ebenfalls als schmale Leiste ausgebildet ist, deren Breite gleich derjenige der Griffplatte (4) ist, und deren die erweiterte Fingerauflage bildende Länge größer als diejenigen der Griffplatte (4) ist.

6. Spritze nach Anspruch 5, **dadurch gekennzeichnet, dass** die über die Griffplatte (4) hinausragenden Enden der Fingerauflagenleiste abgerundet sind.

## Claims

1. Medical syringe, consisting of
a syringe barrel (1) made of glass or plastic, the syringe barrel being formed with a syringe head (1a) in which either a hollow syringe needle is integratable or which has a Luer connection cone, and with a grip plate (4) at another open end of the syringe barrel;
an elastomeric piston stopper being insertable in the syringe barrel (1) and provided with a threaded blind hole (8) for screw connection with a piston rod, the piston rod having a cruciform cross-section; **characterized by**
a plastic part (2) attachable to the grip plate (4) from the syringe head (1a);
wherein the plastic part (2) comprises a retaining section for holding to the syringe barrel and a hood-shaped section (5) enlarged in relation to the grip plate (4) so as to form an enlarged finger support and two one-piece snap catches (3) formed on opposite sides of the hood-shaped section in one piece therewith at an open End of the syringe barrel (1), so that, when the plastic part is attached over the grip plate (4), the snap catches move and snap on an interior side of the syringe barrel (1) with low depth so that a stop is formed preventing unintended withdrawal of the piston stopper and preventing rotation of the piston rod through an angle of more than 90°.

2. Syringe according to claim 1, **characterized in that** the plastic part (2) is formed in one piece.

3. Syringe according to claim 1, **characterized in that** the plastic part (2) comprises several individual components.

4. Syringe according to claims 2 or 3, **characterized in that** the plastic part (2) the individual components are an injection molded part.

5. Syringe according to one of the claims 1 to 4, **characterized in that** the grip plate (4) is strip-shaped and the hood-shaped section of the plastic part (2) enlarged in relation to the grip plate is also strip-shaped and has a width equal to that of the grip plate and said hood-shaped section forming the enlarged finger support has a length that is larger than that of the grip plate (4).

6. Syringe as defined in claim 5, wherein opposite ends of the finger support, which extend beyond said grip plate (4), are rounded.

## Revendications

1. Seringue médicale, se composant de:
- un cylindre de seringue (1) en verre ou en matière plastique, avec une tête de seringue façonnée (1a), dans laquelle soit une canule d'injection est intégrée soit elle comprend un cône de raccordement Luer et avec une plaque de prise (4) à l'extrémité ouverte, ainsi que
- un bouchon piston élastomère, qui peut être introduit dans le cylindre de seringue et qui présente un trou borgne fileté pour le vissage d'une tige de piston ayant une section transversale en croix,
**caractérisée en ce qu'**il est prévu une pièce de matière plastique (2) pouvant être engagée sur la plaque de prise (4) à partir de la tête (1a) du cylindre de seringue (1), qui présente une partie destinée au support sur le cylindre de seringue (1) et une partie en forme de hotte (5) s'évasant en direction de la plaque de prise (4) en formant un appui de doigts agrandi, sur laquelle deux crochets d'encliquetage (3) sont formés d'une seule pièce l'un en face de l'autre dans la région de l'extrémité ouverte du cylindre de seringue, de telle manière qu'ils se glissent au-dessus de la plaque de prise (4) lors de l'engagement et qu'ils s'accrochent sur le côté intérieur du cylindre de seringue (1) avec une faible profondeur de pénétration, de telle manière qu'un élément de butée destiné à empêcher une extraction intempestive du bouchon piston soit ainsi formé et qu'une rotation de la tige de piston au-delà de 90° soit empêchée.

2. Seringue selon la revendication 1, **caractérisée en ce que** la pièce de matière plastique (2) est réalisée d'une seule pièce.

3. Seringue selon la revendication 1, **caractérisée en ce que** la pièce de matière plastique (2) se compose de plusieurs composants individuels.

4. Seringue selon la revendication 2 ou 3, **caractérisée en ce que** la pièce de matière plastique (2) ou le composant individuel respectif est une pièce moulée par injection.

5. Seringue selon l'une quelconque des revendications 1 à 4, **caractérisée en ce que** la plaque de prise (4) est réalisée sous la forme d'une barrette étroite et la partie (5) de la pièce de matière plastique (2) s'évasant en forme de hotte vers la barrette de prise est également réalisée sous la forme d'une barrette étroite, dont la largeur est égale à celle de la plaque de prise (4), et dont la longueur formant l'appui de doigts élargi est plus grande que celle de la plaque de prise (4).

6. Seringue selon la revendication 5, **caractérisée en ce que** les extrémités de la barrette d'appui des doigts saillantes au-delà de la plaque de prise (4) sont arrondies.
